Europäisches Patentamt

**European Patent Office**

Office européen des brevets

(19)

(11) Numéro de publication: **0 061 402**
**B1**

(12) **FASCICULE DE BREVET EUROPEEN**

(45) Date de publication du fascicule du brevet:
03.10.84

(21) Numéro de dépôt: **82400505.2**

(22) Date de dépôt: **19.03.82**

(51) Int. Cl.³: **B 65 B 9/02,** B 65 B 15/04,
A 61 H 39/08, B 65 D 85/24

(54) **Procédé de présentation et d'emballage d'aiguilles d'acupuncture, machine pour la mise en oeuvre de ce procédé et aiguilles d'acupuncture à usage unique.**

(30) Priorité: **20.03.81 FR 8105569**

(43) Date de publication de la demande:
**29.09.82 Bulletin 82/39**

(45) Mention de la délivrance du brevet:
**03.10.84 Bulletin 84/40**

(84) Etats contractants désignés:
**CH DE GB IT LI**

(56) Documents cités:
**FR - A - 2 365 274**
**US - A - 2 074 562**

(73) Titulaire: **Tran Dinh, Can, 20 Avenue de Friedland,**
**F-75008 Paris (FR)**

(72) Inventeur: **Tran Dinh, Can, 20 Avenue de Friedland,**
**F-75008 Paris (FR)**

(74) Mandataire: **Lerner, François, 5, rue Jules Lefebvre,**
**F-75009 Paris (FR)**

ACTORUM AG

## Description

La présente invention a pour objet des aiguilles d'acupuncture et se rapporte plus spécifiquement à la présentation et à l'emballage de telles aiguilles, à usage unique, pouvant être jetées après emploi. Sauf cas particuliers dans lesquels on peut utiliser des aiguilles à pointe en or ou en argent, les aiguilles d'acupuncture utilisées couramment aujourd'hui sont constituées de l'aiguille proprement dite en acier montée sur un manche. L'aiguille doit être fine et très pointue. Après usage, elle doit être soigneusement désinfectée avant toute réutilisation. Evidemment, toute aiguille émoussée ou tordue doit être éliminée.

L'objet de l'invention est de faciliter l'usage des aiguilles par le médecin acupuncteur, en le déchargeant des tracas de l'aseptisation, en mettant à sa disposition chaque fois de nouvelles aiguilles en parfait état, et en facilitant en outre sa prise en main de plusieurs aiguilles. En effet, il est fréquent que le médecin ait à placer sur un même patient plusieurs aiguilles, et fréquemment plus de trois. Dans ces conditions, le médecin acupuncteur est obligé de tenir d'une main plusieurs aiguilles en attente, tandis que de l'autre il place les aiguilles une à une, l'opération étant compliquée par le fait que les aiguilles en attente doivent rester aseptiques et ne peuvent donc être tenues que par le manche relativement petit.

Selon l'invention, les difficultés d'emploi et d'aseptisation susmentionnées sont évitées par le fait que les aiguilles se présentent sous la forme d'un chapelet d'aiguilles parallèles dont la pointe et la plus grande partie de l'aiguille sont enserrées de façon étanche entre deux bandes d'emballage étanche et dont les manches dépassent en dehors de l'emballage. Il apparaît immédiatement que, avec une telle présentation, l'acupuncteur pourra sans difficulté et sans précaution aucune tenir dans sa main un chapelet d'aiguilles aseptiques dont il ne détachera, juste avant sa mise en place, que l'aiguille dont il aura besoin. Bien entendu, ces aiguilles seront jetées après usage. On notera que la matière utilisée, bien que noble, est très limitée au niveau du poids, de sorte que le prix de revient de telles aiguilles fabriquées en grande série pourra rendre l'usage de telles aiguilles économique, eu égard à l'économie d'aseptisation (matériel et main-d'œuvre) qu'elles permettront de réaliser. En outre, du point de vue de la commodité et de la sécurité d'emploi, elles seront bien préférables.

Dans la mise en œuvre de l'invention, les aiguilles seront présentées disposées en chapelet parallèlement les unes aux autres, avec la pointe et la plus longue partie au moins des aiguilles enveloppées de façon étanche et aseptique entre deux bandes continues d'un papier, d'un film en plastique ou analogue, de protection, au moins la plus grande partie du manche de l'aiguille faisant saillie hors desdites bandes de protection.

L'invention se rapporte également à une machine permettant l'emballage d'aiguilles d'acupuncture comme indiqué ci-dessus, ladite machine étant caractérisée en ce qu'elle comporte:

— une bande continue d'alimentation des aiguilles parallèlement les unes derrière les autres;

— des moyens de déroulement d'un film en plastique, en papier ou analogue, sur ladite bande, ledit film couvrant une grande partie de l'aiguille et dépassant sa pointe;

— des moyens de déroulement par-dessus le film en plastique, en papier ou analogue, d'une autre bande d'un film, en papier ou analogue, de largeur sensiblement identique, et

— des moyens de soudage des deux bandes par chauffage et pression l'une contre l'autre, en enserrant entre elles de façon étanche la plus grande partie de l'aiguille, et plus particulièrement sa pointe.

L'invention apparaîtra plus clairement à l'aide de la description qui va suivre, faite en référence aux dessins annexés dans lesquels:

la fig. 1 montre schématiquement, vue selon la flèche I de la fig. 2, une partie de la bande d'entraînement des aiguilles dans la machine;

la fig. 2 est une vue en coupe faite sensiblement selon le plan II-II de la fig. 1;

la fig. 3 est une vue de dessus faite selon la flèche III de la fig. 1;

la fig. 4 montre en vue schématique une machine permettant l'emballage des aiguilles conformément à l'invention;

la fig. 5 montre à plus grande échelle, au niveau du détail entouré V dans la fig. 2, comment se présente une aiguille d'acupuncture sur sa bande de convoyage lorsque les deux films d'emballage sont appliqués de part et d'autre de l'aiguille, et

la fig. 6 montre un fragment d'un chapelet d'aiguilles emballées conformément à l'invention.

Selon le procédé et la machine d'emballage illustrés aux dessins, les aiguilles d'acupuncture, comprenant l'aiguille proprement dite 1 avec leur manche de préhension 2 à l'opposé de la pointe 3, sortent de la machine toutes emballées en un chapelet (fig. 6) dont la continuité est assurée par la bande continue d'emballage 4 qui enveloppe de part et d'autre la partie 1 des aiguilles. La bande d'emballage 4 dépasse nettement au-delà de la pointe 3 des aiguilles. Par contre, elle s'arrête de façon à laisser sensiblement tout le manche 2 de l'aiguille découvert.

La bande 4 d'emballage est en fait constituée de deux couches, à savoir une couche 5 inférieure et une couche 6 supérieure, qui sont collées l'une contre l'autre et qui enferment de façon étanche et aseptique les parties 1 des aiguilles.

L'emballage peut se faire en continu en approvisionnant les aiguilles nues sur un convoyeur 7, constitué par exemple par un tapis caoutchouté, entraîné en continu, comme indiqué par les flèches, par des rouleaux d'entraînement 8, 9 de la machine. La bande 7 comporte une série de cannelures en creux 10, de dimension suffisante pour loger sensiblement la moitié du manche 2 d'une aiguille. Les parties en creux 10 assurent le bon positionnement et le bon espacement tant longitudinal que transversal des aiguilles parallèlement les unes aux autres sur la bande 7. Les aiguilles peuvent être approvisionnées à partir d'une trémie 11 et l'on peut prévoir tout dispositif automatique ou manuel pour éliminer des aiguilles qui ne seraient pas bien positionnées, ou pour combler un emplacement éventuellement laissé libre.

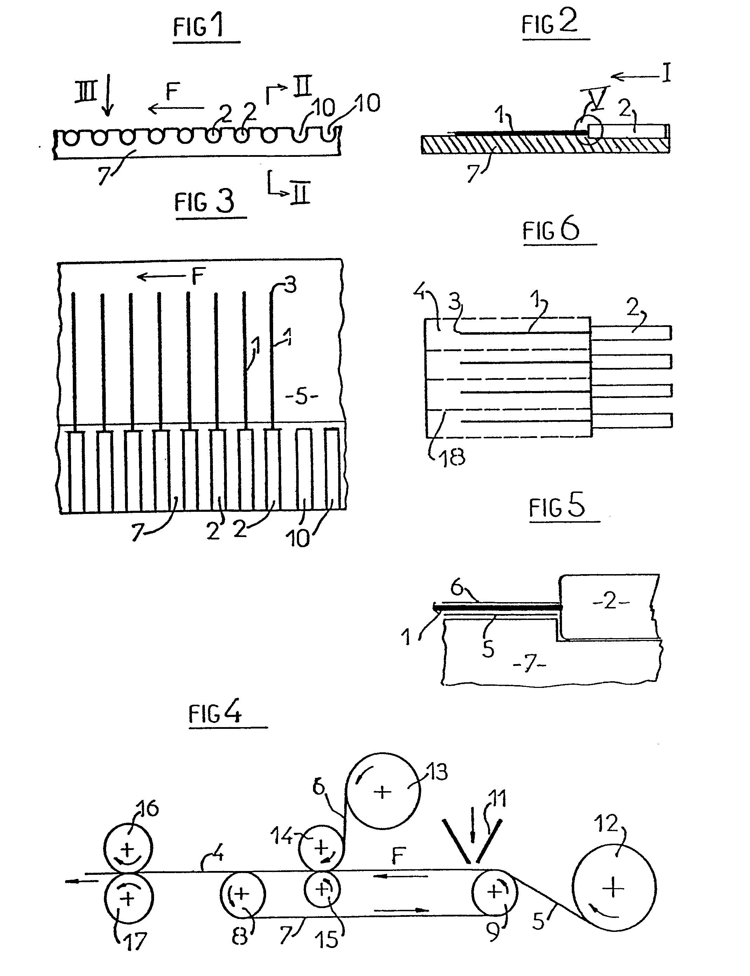

En même temps que le convoyeur 7 avance en emmenant les aiguilles selon la flèche F, la bande d'emballage inférieure 5 est déroulée à partir d'un rouleau d'approvisionnement 12 à la même vitesse que le convoyeur 7 et sur une largeur intéressant sensiblement toute la longueur de la partie 1 formant l'aiguille proprement dite (fig. 3). De la même façon, la feuille d'emballage supérieure 6 est déroulée et amenée à partir d'un rouleau approvisionneur 13. Les deux feuilles 5 et 6 sont pressées entre deux rouleaux 14, 15 qui avancent à la même vitesse que celle de défilement du tapis 7. Ces rouleaux 14, 15 sont avantageusement chauffants, de sorte qu'ils réalisent non seulement l'entraînement des bandes 5, 6, mais leur soudure thermique. Dans ce cas, la nature des matériaux constitutifs des bandes 5, 6 est évidemment choisie de façon à permettre cette soudure et assurer ensuite le maintien de la qualité aseptique de l'emballage.

L'aseptisation peut être faite du seul fait de la soudure à chaud des deux films, ou elle peut être parfaite par d'autres moyens tels que le passage du chapelet dans un dispositif générateur de rayons ultraviolets ou de rayons γ.

Avantageusement, à la sortie du dispositif, on prévoit deux rouleaux supplémentaires 16, 17 crantés tournant en synchronisme avec l'avancement de la bande et formant dans l'emballage des prédécoupes, telles que visibles en 18 à la fig. 6, qui faciliteront le détachement des aiguilles une par une du chapelet.

Les chapelets d'aiguilles pourront être ensuite groupés dans des petits cartonnages en quantité convenable, par exemple de 20, 50 ou 100 aiguilles.

Au moment de l'utilisation, le praticien découpera une petite longueur de chapelet et pourra ainsi tenir d'une seule main, sans difficulté, une réserve d'aiguilles neuves, aseptiques, prêtes à l'emploi. Il lui suffira de prélever les aiguilles au fur et à mesure de ses besoins.

Bien entendu, l'invention n'est nullement limitée au mode de réalisation et de mise en œuvre illustré qui n'a été donné qu'à titre d'exemple, l'invention comprenant au contraire tous les équivalents techniques des moyens décrits ainsi que leurs combinaisons, si celles-ci sont réalisées suivant son esprit et mises en œuvre dans le cadre des revendications qui suivent.

**Revendications**

1. Procédé de présentation et d'emballage d'aiguilles (1) d'acupuncture, caractérisé en ce qu'il consiste à présenter les aiguilles (1) disposées en chapelet parallèlement les unes aux autres, avec la pointe et la plus longue partie au moins des aiguilles (1) enveloppées de façon étanche et aseptique entre deux bandes continues (5, 6) d'un papier, d'un film en plastique ou analogue, de protection, au moins la plus grande partie du manche (2) de l'aiguille faisant saillie hors desdites bandes de protection (5, 6).

2. Procédé selon la revendication 1, caractérisé en ce qu'on ménage dans lesdites bandes des amorces (18) de déchirure entre deux aiguilles (1) contiguës.

3. Machine pour l'emballage d'aiguilles d'acupuncture (1) selon le procédé de présentation et d'emballage de l'une des revendications 1 ou 2, caractérisée en ce qu'elle comporte:
— une bande continue (7) d'alimentation des aiguilles (1) parallèlement les unes derrière les autres;
— des moyens de déroulement d'un film (5) en plastique, en papier ou analogue sur ladite bande (7), ledit film couvrant une grande partie de l'aiguille (1) et dépassant sa pointe (3);
— des moyens de déroulement, par-dessus le film en papier ou analogue, d'une autre bande d'un film en plastique, en papier ou analogue (6), de largeur sensiblement identique, et
— des moyens de soudage (14, 15) des deux films (5, 6) par chauffage et pression l'un contre l'autre, en enserrant entre eux de façon étanche la plus grande partie de l'aiguille (1), et plus particulièrement sa pointe (3).

4. Machine selon la revendication 3, caractérisée en ce qu'il est prévu des moyens supplémentaires d'aseptisation par chauffage, rayonnement UV ou γ, notamment à la sortie de la machine du chapelet d'aiguilles (1) emballées.

5. Machine selon l'une des revendications 3 ou 4, caractérisée en ce qu'il est prévu des moyens tels que deux rouleaux (16, 17) assurant le prédécoupage du chapelet entre deux aiguilles voisines.

6. Machine selon l'une des revendications 3 à 5, caractérisée en ce que la bande continue présente des parties en creux (10) susceptibles de loger sensiblement la moitié du manche (2) de l'aiguille (1).

7. Aiguilles pour acupuncture à usage unique, caractérisées en ce qu'elles se présentent sous la forme d'un chapelet d'aiguilles parallèles dont la pointe (3) et la plus grande partie de l'aiguille (1) sont enserrées de façon étanche entre deux bandes d'emballage (5, 6) étanches, et dont les manches (2) dépassent en dehors de l'emballage.

**Claims**

1. Method of presenting and packing acupuncture needles, characterized in that it consists in presenting the needles (1) in a row, parallel to one another, with the point and the longest part of the needles (1) at least wrapped in an airtight and sterile manner between two continuous strips (5, 6) of a paper, plastic film, or similar protective strip, with at least the majority of the handle (2) of the needle protruding beyond the said protective strips (5, 6).

2. Method according to Claim 1, characterized in that there are serrations (18) cut into the said bands between two consecutive needles (1).

3. Machine for the packing of acupuncture needles (1) in accordance with the presenting and packing method of one of Claims 1 or 2, characterized in that it comprises:
— a continous strip (7) for feeding the needles (1) in parallel, one after the other;
— devices for unrolling a plastic, paper or analogous film (5) on the said strip (7), said film covering a majority of the needle (1) and extending beyond its tip (3);
— devices for unrolling, atop the film, paper or analogous film, another strip of a plastic, paper or analogous film (6), whose width is roughly the same, and
— means of bonding (14, 15) the two films (5, 6) by means of heating and pressure, sealing the largest portion of the needle (1) between them, particularly its point (3), in an airtight manner.

4. Machine according to Claim 3, characterized in that additional sterilization devices are provided which function by heating, ultraviolet, or γ radiation, especially at the output end of the machine for the rows of packed needles (1).

5. Machine according to one of Claims 3 or 4, characterized in that devices are provided such as two rollers (16, 17) which provide for the precutting of the row between two adjacent needles.

6. Machine according to one of Claims 3 to 5, characterized in that the continuous strip features hollowedout parts (10) which are capable of housing roughly half the handle (2) of the needle (1).

7. Acupuncture needles for single use, characterized in that they are arranged in parallel in rows in which their points (3) and the majority of the needle (1) are wrapped in an airtight manner between two airtight packing strips (5, 6) and their handles (2) protrude outside the packing.

## Patentansprüche

1. Verfahren zum Präsentieren und Verpacken von Akkupunkturnadeln (1), dadurch gekennzeichnet, dass die Nadeln (1) in Reihe und parallel zueinander angeordnet präsentiert werden, wobei die Spitze und mindestens der längste Teil der Nadeln (1) in dichter und aseptischer Weise zwischen kontinuierlichen Schutzbändern (5, 6) aus Papier, Kunststoffilm od. dgl. eingehüllt sind und mindestens der grösste Teil des Griffes (2) der Nadel einen Vorsprung aus den Schutzbändern (5, 6) heraus bildet.

2. Verfahren nach Anspruch 1, dadurch gekennzeichnet, dass man die Bänder zwischen zwei benachbarten Nadeln (1) mit Einrichtungen (18) zum Aufreissen versieht.

3. Maschine zum Verpacken von Akkupunkturnadeln (1) nach dem Verfahren zum Präsentieren und Verpacken gemäss einem der beiden Ansprüche 1 oder 2, dadurch gekennzeichnet, dass sie aufweist:
— ein kontinuierliches Band (7) zum Zuführen der Nadeln (1) parallel und die einen hinter den anderen,
— Mittel zum Abrollen eines Filmes (5) aus Kunststoff, Papier od. dgl. auf dem Band (7), wobei dieser Film einen grossen Teil der Nadel (1) abdeckt und über ihre Spitze (3) hinausgeht,
— Mittel zum Abrollen eines anderen Bandes eines Kunststoffilmes, Papiers od. dgl. (6), der im wesentlichen die gleiche Länge hat, oberhalb des Filmes, Papiers od. dgl., und
— Mittel zum Verschweissen (14, 15) der zwei Filme (5, 6) durch Erhitzen und Druck des einen gegen den anderen, unter Einschliessen des grössten Teils der Nadel (1), und insbesondere ihrer Spitze (3) dichtend zwischen diesen Filmen.

4. Maschine nach Anspruch 3, dadurch gekennzeichnet, dass sie mit zusätzlichen Mitteln zum Sterilisieren der Reihe der verpackten Nadeln (1) versehen ist, durch Erhitzen, UV-Bestrahlen oder γ-Bestrahlung, insbesondere am Ausgang der Maschine.

5. Maschine nach einem der beiden Ansprüche 3 oder 4, dadurch gekennzeichnet, dass sie mit Mitteln versehen ist, wie z.B. zwei Walzen (16, 17), welche das Vorschneiden der Reihe zwischen zwei benachbarten Nadeln sicherstellen.

6. Maschine nach einem der Ansprüche 3 bis 5, dadurch gekennzeichnet, dass das kontinuierliche Band vertiefte Teile (10) aufweist, die in der Lage sind, im wesentlichen die Hälfte des Griffes (2) der Nadel (1) aufzunehmen.

7. Akkupunkturnadeln zum einmaligen Gebrauch, dadurch gekennzeichnet, dass sie sich in Form einer Reihe paralleler Nadeln präsentieren, deren Spitze (3) und deren grösster Teil der Nadel (1) dichtend zwischen zwei dichten Verpackungsbändern (5, 6) eingeschlossen sind und deren Griffe (2) aus der Verpackung herausragen.

FIG 1

FIG 2

FIG 3

FIG 6

FIG 5

FIG 4